(19) Europäisches Patentamt
European Patent Office
Office européen des brevets

(11) Publication number: **0 167 651**
**B1**

(12) # EUROPEAN PATENT SPECIFICATION

(45) Date of publication of patent specification: **13.09.89**

(21) Application number: **84108148.2**

(22) Date of filing: **11.07.84**

(51) Int. Cl.⁴: **C 07 D 501/18,** C 07 D 501/12, C 12 P 35/00, C 07 D 501/36

(54) **Process for producing 3-cephem-4-carboxylic acid derivatives substituted in 3-position.**

(43) Date of publication of application:
**15.01.86 Bulletin 86/03**

(45) Publication of the grant of the patent:
**13.09.89 Bulletin 89/37**

(84) Designated Contracting States:
**AT CH DE FR GB IT LI NL**

(56) References cited:
**GB-A-2 048 257**
**US-A-3 979 383**

**The file contains technical information submitted after the application was filed and not included in this specification**

(73) Proprietor: **Antibioticos, S.A.**
**Calle Bravo Murillo, no. 38**
**28003 Madrid (ES)**

(72) Inventor: **Lizarbe, D. Jose Ramon Fernandez, Dr.**
**C/ Artesa de Segre, 12**
**28035-Madrid (ES)**
Inventor: **Alba, D. Alejandro Vitaller, Dr.**
**C/ Del Carmen, 3**
**24001-Leon (ES)**

(74) Representative: **Vossius & Partner**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 167 651 B1**

**Description**

This invention relates to a valuable method for the purification of compounds with the "cephem" structure substituted in the 3 position, which are used as starting products in the synthesis of substances so much appreciated in the pharmaceutical field as the so-called cephalosporins of "second" and "third" generation:

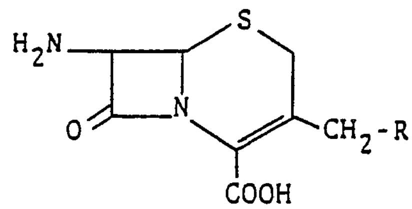

Basically the process for producing 3-cephem-4-carboxylic acid derivatives substituted in 3-position is carried out according to the following scheme, in which a molecule of acetic acid is liberated; see US—A—3,979,383:

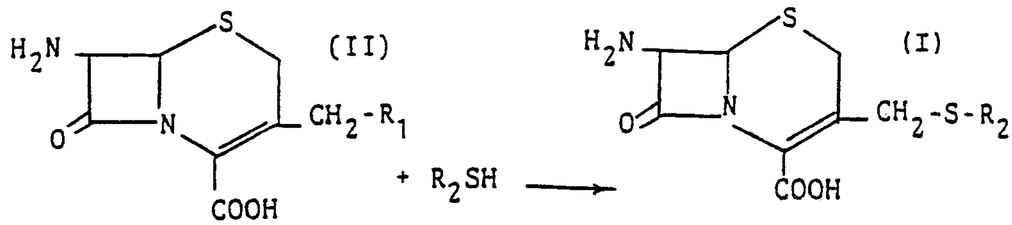

(II) (I)

where $R_1$ means the $—OOCCH_3$ group and $R_2$ is thienyl, diazolyl, triazolyl, tetrazolyl, thiazolyl, thiadiazolyl, thiatriazolyl, oxazolyl, oxadiazolyl, pyridyl, pyrimidinyl, benzothiazolyl, benzimidazolyl, benzoxazolyl, or their substituted derivatives in any of the possible positions of the mentioned heterocyclic structure, available for substitution.

The important aspect of the process is the fact that a highly selective purification procedure is utilized, which allows to obtain in a single step substances of adequate quality for a subsequent use by the pharmaceutical industry.

It is well-known by the experts in the art that the use of impure intermediate products renders the purification of the finally synthetized cephalosporins for reaching an adequate degree of quality compatible with the pharmaceutical requirements extraordinarily difficult. Therefore it is necessary to take care of such complex problems as:

— Level of unreacted starting materials
— Separation of degradation products and products of side reactions from the synthesis itself, for example the $\Delta^2$ isomer obtained by shift of the double bond existing between the positions 3 and 4 of the "cephem" structure to the position 2—3 (IV), the desacetyl-lactone, formed by internal cyclization of the carboxyl of the position 4 with the substituent of the position 3 (V).

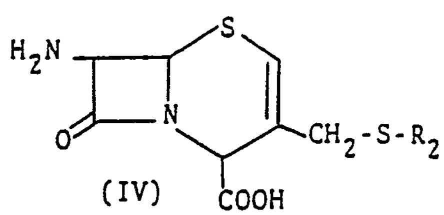

(IV)

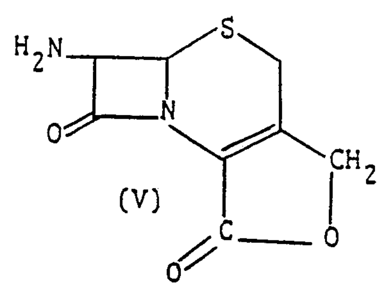

(V)

and of other degradation products, not absolutely identified, coloured or not, which are a serious problem in the subsequent production steps.

It is the object of the invention to provide a novel process for producing in an excellent degree of purity 3-cephem-4-carboxylic acid derivatives substituted in 3-position which are valuable intermediates in the synthesis of therapeutically useful cephalosporins.

This object is solved by the surprising finding that by performing a step of adsorption-elution using a macroreticular strongly basic anion exchange resin, an excellent degree of purity can be obtained.

The subject matter of the invention is a process for producing a compound of the general formula I

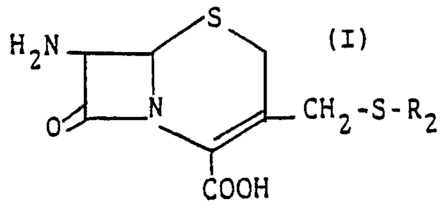

(I)

wherein $R_2$ is thienyl, diazolyl, thiazolyl, tetrazolyl, thiadiazolyl, thiatriazolyl, oxazolyl oxadiazolyl, pyridyl, pyrimidinyl, benzothiazolyl, benzimidazolyl, benzoxazolyl or any of their substituted derivatives in any of the possible positions of the mentioned heterocyclic residues available for substitution, comprising

a) reacting in an aqueous medium, at a pH between 5,0 and 8,0 and at a temperature between 60°C and 80°C a 7-aminocephalosporanic acid (7—ACA) derivative of the general formula II

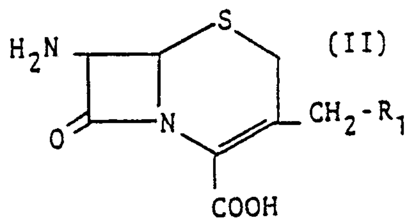

(II)

wherein $R_1$ means $—OOCCH_3$ with a thiol compound of the general formula III

$R_2—SH$ (III)

wherein $R_2$ is as defined above, and

b) purifying the reaction product obtained in the previous step by means of adsorption over an anion exchange resin having a crosslinked, acrylic copolymer structure and containing functional quaternary ammonium groups, characterized in that the anion exchange resin used in step b) is a macroreticular, strongly basic resin.

A particular advantage of the process of the invention is the fact that synthesis and purification are carried out in aqueous medium. Thus, the inconvenience of a content of residual solvents in the obtained products, which leads to bad consequences regarding the stability, especially long term stability, and imposes decomposition of the pharmaceutically active compounds, is obviated.

The 7-aminocephalosporanic acid (7—ACA), also known as 7-amino-3-cephem-4-carboxylic acid is reacted with a compound from the group of the $R_2SH$ thiols, where $R_2$ means, as previously mentioned, thienyl, diazolyl, triazolyl, tetrazolyl, thiazolyl, thiadiazolyl, thiatriazolyl, oxazolyl, oxadiazolyl, pyridyl, pyrimidinyl, benzothiazolyl, benzimidazolyl, benzoxazolyl, or with a derivative thereof, especially alkyl substituted derivatives, wherein the substituent may be in any position of the heterocyclic structure which is available.

The process is a general one and is carried out in an aqueous medium, dissolving the 7-aminocephalosporanic acid and the corresponding thiol in the vehicle of the reaction by addition of an alkali metal hydroxide, ammonium hydroxide or basic compounds which forms water soluble salts; evidently the 7-aminocephalosporanic acid and the thiols can be transformed into soluble salts by reacting them with an alkali metal carbonate or bicarbonate, an ammonium salt or in general with any salt which can be used for this reaction, without changing the fundamentals of this process.

A second way can be the direct use of the soluble salts of the 7—ACA and thiols, e.g. the sodium salts if commercially available, and this does not change the essentials of the process.

In a general way the process is carried out by preparing an aqueous solution of 7-aminocephalosporanic acid and the of the respective thiol, in separate recipients or jointly and heating the mixture to an elevated temperature to achieve the condensation in the position 3 of the "cephem" structure and obtaining the 7-amino-3-(substituted)-3-cephem-4-carboxylic acids (I).

The variables of the reaction which should preferably be controlled are the following:

— Reaction concentration: is a variable considered slightly critical and generally concentrations between 3 and 10 per cent are preferred.

— A second important variable is the $R_2SH$/7—ACA molar ratio and the values which can be utilized are between 1,0 and 2,0, preferably between 1,1 and 1,4 mol $R_2SH$ per mol of 7—ACA.

— The conditions of relative acidity in which the reaction takes place are considered as a third variable, which is limited by the stability of the β-lactam ring of the 7—ACA and of the 7-amino-3-(substituted)-3-cephem-4-carboxylic acid product of the reaction and by the need of maintaining the reactants in solution. The adequate pH values are those comprised between 5 and 8 and preferably between 6,5 and 7,5.

The pH of the reaction mixture may be controlled within the mentioned limits by previous adjustment, or in some cases in which it is modified during the condensation by continuous addition of alkali or by use

of buffer solutions; usually the previous adjustment is enough since in the condensation there is no sensible modification the pH of the reaction mixture which is already buffered by itself.

The time and temperature variables present the normal tendencies to accelerate the reaction at a high temperature, and for practical reasons reaction times from 0.5 up to 6 hours preferably between 1 and 2 hours, and temperatures from 60 up to 80°C are preferred.

Once finished the reaction period the reaction mixture is cooled and acidified up to pH values between 2 and 3,5 preferably with strong mineral acids, such as hydrogen halides or oxy acids. A crude product is precipitated which can be subjected to the subsequent purification step.

Equally acceptable are two other variants: a procedure according to which the product of the previous reaction, at a pH between 6,5 and 7,5 is adsorbed over the ionic exchange resin directly, without the mediation of the crude compound precipitation step, or a procedure according to which the precipitated compound, as mentioned in the above paragraph, is suspended in water to be again dissolved by addition of the quantities of alkali needed to bring the solution to the adequate pH which oscillates from 6,5 up 8,5.

It has been found that a macroreticular, strongly basic anion exchange resin having a crosslinked acrylic copolymer structure and containing functional quaternary ammonium groups is preferred in the sense that it produces practically quantitive results and a high degree of purification.

The quantity of the resin is based on the consideration that the theoretical exchange capacity of the mentioned resin is in the order of 1—1,2 meq/ml. Depending on the height/diameter ratio of the column and on the degree of the tolerated losses, a quantity of between 3 and 10 ml of resin/gram of compound to be adsorbed will be utilized.

The resin is used in the acetate state which may be obtained — following the indications of the manufacturer to convert it to the free base state — by treating it in re-cycle with a quantity of acetic acid in the order of 1,2—1,5 equiv/l of resin, for assuring a total conversion to the acetate state.

Right after this the resin is carefully washed and adjusted in batch to the pH required for the posterior adsorption by addition of sodium hydroxyde.

Then the solution to be purified is passed over the pre-treated resin at such flows that the time of contact is between 20 and 40 minutes, in descending flow. Finished the adsorption, the column is displaced with 2—3 volumes of water.

The use of an ionic exchange resin allows the elimination of non-ionic impurities which might be present (lactone type), which are hardly eliminated by any other procedure.

Among the different types of eluents the use of a sodium acetate buffer solution with a concentration of 0,1—0,5 M and a pH of 7—7,5 is preferred.

From the eluate so obtained, the product of the invention is isolated according to conventional acidification methods.

The following examples illustrate the present invention.

Example No. 1

32 g of 2-methyl-5-mercapto-1,3,4-thiadiazol were dissolved in 350 ml of water, adjusting the pH at 6,8 with ammonium hydroxyde and the solution was heated to 75°C. Simultaneously 51 g of 7—ACA were dissolved in 720 ml of water by addition of ammonium hydroxyde up to pH 7,5. The solutions were mixed-up, the temperature of the mixture was adjusted to 78°C and the pH to 6,0—7,0, keeping the mentioned conditions for 90 minutes. It was cooled quickly to 25°C and was crystallized by adjusting the pH to 2,5 by addition of hydrochloric acid. The crystallization was prolonged for 1 hour of stirring at 0,5°C. The obtained compound was filtrated and washed with 250 ml of water and 3 portions of 250 ml of 80% acetone each one. By this way 51,4 g of the 7-amino-3(2-methyl-5-mercapto-1,3,4-thiadiazolyl)-3-cephem-4-carboxylic acid were obtained. 50 g of this compound were dissolved in 1 l of water by addition of ammonium hydroxyde up to pH 7,5. This solution was passed through a column loaded with 500 ml of AMBERLITA IRA—958® resin (manufactured by Rohm and Haas) in the acetate cycle, at a flow of 20 ml/min, discarding the efluent. It was eluted with sodium acetate buffer 0,4 M, pH 7,5 at a flow of 13 ml/min, retaking the central fraction of eluate, considering as such that in which precipitate appears upon acidification, obtaining 1.460 ml of eluate. From this eluate which contains the 7-amino-3(2-methyl-5-mercapto-1,3,4-thiadiazolyl)-3-cephem-4-carboxylic acid purified and in the form of sodium salt, the compound was crystallized in acid form by adjusting the pH at 2,5 by addition of concentrated hydrochloric acid. It was filtered, washed, and dried according to the conventional procedures, obtaining 40.8 g of compound with a purity higher than 96 percent.

Identification, IR (KBr) $cm^{-1}$, 1.803, 1.618;

3,35 (1H, d, $J_{HbHd}$ = 9,3 Hz)
3,74 (1H, d, $J_{HaHa}$ = 12,45 Hz)
3,86 (1H, d, $J_{HdHb}$ = 9,3)
4,44 (1H, d, $J_{HcHa}$ = 12,45 Hz)
4,68 (1H, d, $J_{H6H7}$ = 4,8 Hz)
4,95 (1H, d, $J_{H2H6}$ = 4,8 Hz)

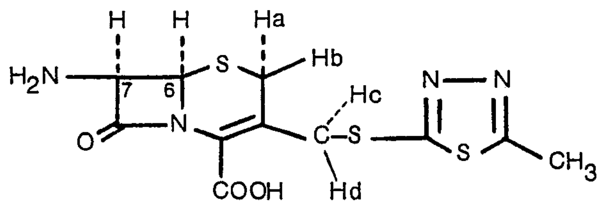

Example No. 2

12,76 g of 1-methyl-5-mercapto-1,2,3,4-tetrazol were dissolved in 300 ml of water, being adjusted the pH at 6,6 by means of ammonium hydroxyde and the solution was heated to 70°C. Simultaneously 29,6 g of 7—ACA were dissolved in 400 ml of water by addition of ammonium hydroxyde up to pH 7,5. The mentioned solutions were mixed up and the stirring was kept for 70 minutes at 68°C, adding the ammonium hydroxyde necessary to keep the pH between 6,8 and 7,0. It was quickly cooled to 25°C and the pH was adjusted at 2,5 with concentrated hydrochloric acid. Immediately thereafter it was cooled to 0—5°C and the crystallization was performed for 1 hour with stirring. The compound was filtered and washed with 12 ml of water and 350 ml of acetone. 24,2 g of 7-amino-3-(1-methyl-5-mercapto-1,2,3,4-tetrazolyl)-3-cephem-4-carboxylic acid were obtained. This crude compound was dissolved in 0,5 l of water by addition of ammonium hydroxyde up to steady pH 7,5. This solution was passed through a column loaded with 250 ml of AMBERLITA IRA—958® (manufactured by Rohm and Haas) in the acetate cycle, at a flow of 20 ml/min., discarding the efluent. The column was displaced with 400 ml of water, at the same flow, discarding the efluent likewise. Then was eluted with sodium acetate buffer 0,4 M, pH 7,5, at a flow of 13 ml/min., taking up the central fraction of the eluate, considering as such that in which a precipitate is formed upon acidifying. 730 ml of eluate were retaken, containing the 7-amino-3-(1-methyl-5-mercapto-1,2,3,4-tetrazolyl)-3-cephem-4-carboxylic acid in the form of sodium salt. The free acid was crystallized by adjusting the pH to 2,5 by addition of concentrated hydrochloric acid. It was filtered, washed and dried according to the conventional procedures, being obtained 20,4 g of compound with a purity higher than 96 percent.

Identification, IR (KBr) $cm^{-1}$, 1.790, 1.610.

NMR ($D_2O+CF_3COOD$) ppm:

3,82 (2H, s, $C_2H_2$)
4,07 (3H, s, N—$CH_3$)
4,41 (1H, d, $J_{HaHb}$ = 13,7 Hz)
4,26 (1H, d, $J_{HbHa}$ = 13,7 Hz)
5,26 (1H, d, $J_{H7}$ = 4,8 Hz)
5,19 (1H, d, $J_{H6}$ = 4,8 Hz)

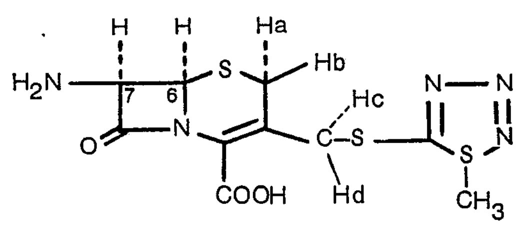

## Claims

1. A process for producing a compound of the general formula I

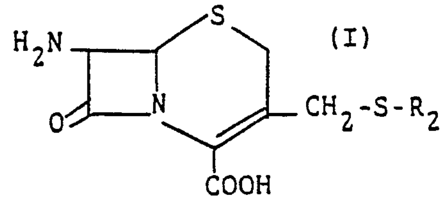

(I)

wherein $R_2$ is thienyl, diazolyl, thiazolyl, tetrazolyl, thiadiazolyl, thiatriazolyl, oxazolyl oxadiazolyl, pyridyl, pyrimidinyl, benzothiazolyl, benzimidazolyl, benzoxazolyl or any of their substituted derivatives in any of the possible positions of the mentioned heterocyclic residues available for substitution, comprising

a) reacting in an aqueous medium, at a pH between 5,0 and 8,0 and at a temperature between 60°C and 80°C a 7-aminocephalosporanic acid (7—ACA) derivative of the general formula II

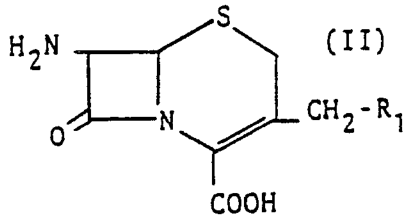

(II)

wherein $R_1$ means —$OOCCH_3$ with a thiol compound of the general formula III

$$R_2—SH \qquad (III)$$

wherein $R_2$ is as defined above, and

b) purifying the reaction product obtained in the previous step by means of adsorption over an anion exchange resin having a crosslinked, acrylic copolymer structure and containing functional quaternary ammonium groups, characterized in that the anion exchange resin used in step b) is a macroreticular, strongly basic resin.

2. A procss according to claim 1, characterized in that ion exchange resin is in the acetate state.

3. A process according to claims 1 and 2, characterized in that the product is eluted from the resin with a sodium acetate buffer solution having a concentration between 0,1 and 0,5 M and a pH between 7 and 7,5.

**Patentansprüche**

1. Verfahren zur Herstellung einer Verbindung der allgemeinen Formel I

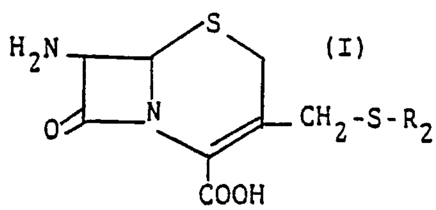

(I)

in der $R_2$ eine Thienyl-, Diazolyl-, Thiazolyl-, Tetrazolyl-, Thiadiazolyl-, Thiatriazolyl-, Oxazolyl-, Oxadiazolyl-, Pyridyl-, Pyrimidinyl-, Benzothiazolyl-, Benzimidazolyl- oder Benzoxazolylgruppe oder irgendeines ihrer substituierten Derivate in irgendeiner der möglichen Stellungen der erwähnten heterocyclischen Reste, die für Substitution verfügbar sind, bedeutet, umfassend

a) Umzetzung in einem wäßrigen Medium bei einem pH-Wert zwischen 5,0 und 8,0 und bei einer Temperatur zwischen 60 und 80°C eines 7-Aminocephalosporansäure (7—ACA)-Derivates der allgemeinen Formel II

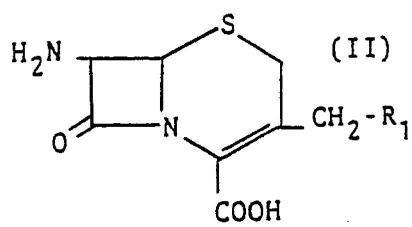

(II)

in der $R_1$ die Gruppe —$OOCCH_3$ bedeutet, mit einer Thiol-Verbindung der allgemeinen Formel III

$$R_2—SH \qquad (III)$$

in der $R_2$ wie vorstehend definiert ist, und

b) Reinigung des in der vorangehenden Stufe erhaltenen Reaktionsproduktes durch Adsorption über einem Anionenaustauscherharz mit einer vernetzten Acrylcopolymerisat-Struktur, das funktionelle quaternäre Ammoniumgruppen enthält, dadurch gekennzeichnet, daß das in Stufe b) verwendete Anionenaustauscherharz ein makroretikuläres, stark basisches Harz ist.

2. Verfahren nach Anspruch 1, dadurch gekennzeichnet, daß das Ionenaustauscherharz im Acetat-Zustand vorliegt.

3. Verfahren nach Anspruch 1 und 2, dadurch gekennzeichnet, daß das Produkt aus dem Harz mit einer Natriumacetat-Pufferlösung eluiert wird, die eine Konzentration zwischen 0,1 und 0,5 M und einen pH-Wert zwischen 7 und 7,5 aufweist.

**Revendications**

1. Un procédé pour produire un composé de formule générale I

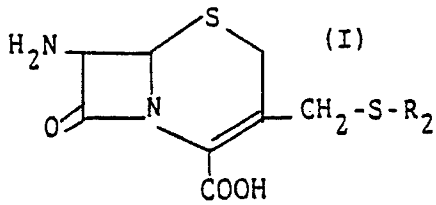

(I)

dans laquelle $R_2$ est un thiényle, un diazolyle, un thiazolyle, un tétrazolyle, un thiadiazolyle, un thiatriazolyle, un oxyazolyle, un oxadiazolyle, un pyridyle, un pyrimidinyle, un benzothiazolyle, un benzimidazolyle, un benzoxazolyle, ou l'un quelconque de leurs dérivés substitués dans l'une quelconque des positions possibles des restes hétérocycliques mentionnés disponibles pour la substitution qui comprend

a) la réaction dans un milieu aqueux à un pH entre 5,0 et 8,0 et à une température entre 60°C et 80°C d'un dérivé d'acide 7-aminocéphalosporanique (7—ACA), répondant à la formule générale II

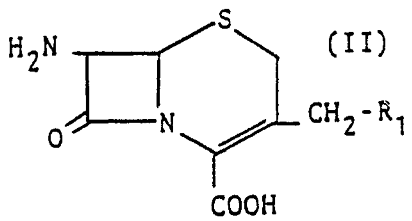

(II)

dans laquelle $R_1$ représente $—OOCCH_3$, avec un thiol répondant à la formule générale III

$$R_2—SH \quad \text{(III)}$$

dans laquelle $R_2$ est comme défini ci-dessus et

b) la purification du produit réactionnel obtenu dans le stade précédent par adsorption sur une résine échangeuse d'anions ayant une structure de copolymère acrylique réticulé et contenant des groupes fonctionnels ammonium quaternaire, caractérisé en ce que la résine échangeuse d'anions utilisée dans le stade b) est un résine fortement basique macroréticulaire.

2. Un procédé selon la revendication 1, caractérisé en ce que la résine échangeuse d'ions est sous la forme acétate.

3. Un procédé selon les revendications 1 et 2, caractérisé en ce que le produit est élué de la résine avec une solution de tampon acétate de sodium ayant une concentration entre 0,1 et 0,5 M et un pH entre 7 et 7,5.